# EUROPÄISCHE PATENTSCHRIFT

(11) **EP 0 555 661 B1**
(45) Veröffentlichungstag und Bekanntmachung des Hinweises auf die Patenterteilung: **05.03.1997**
(21) Anmeldenummer: 93100693.6
(22) Anmeldetag: 19.01.1993
(51) Int. Cl.: C12P 13/08, C12N 1/20, C12N 15/01

(54) **Verfahren zur Herstellung von L-Lysin durch Fermentation von coryneformen Bakterien**
Process for production of L-lysine by fermentation of coryneform bacteria
Procédé de production de L-lysine par fermentation de bactéries coryneformes

(30) Priorität: 14.02.1992 DE 4204361
(43) Veröffentlichungstag der Anmeldung: 18.08.1993
(73) Patentinhaber: Degussa Aktiengesellschaft, 60311 Frankfurt (DE)
(72) Erfinder: Bachmann, Bernd, Dr., D-33824 Werther (DE); Kautz, Sabine, W-4800 Bielefeld 16 (DE); Thierbach, Georg, Dr., W-4800 Bielefeld 1 (DE); Pühler, Alfred, Prof. Dr., W-4800 Bielefeld 15 (DE); Kalinowski, Jörn, Dr., W-4800 Bielefeld 1 (DE)

(56) Entgegenhaltungen:
- DE-A- 4 023 576
- US-A- 3 711 374
- US-A- 4 411 997
- US-A- 4 626 504
- JOURNAL OF BACTERIOLOGY, Band 172, Nr. 3, März 1990, Baltimore, MD (US); A. SCHEFER et al., S. 1663-1666

## Beschreibung

Die Erfindung betrifft ein Verfahren zur fermentativen Herstellung von L-Lysin, bei dem man coryneforme Bakterien einsetzt, die aufgrund einer Insertionsmutagenese eine defekte Homoserin Dehydrogenase besitzen und eine Homoserin Auxotrophie zeigen.
Die essentielle Aminosäure L-Lysin ist als Nahrungs- und Tierfutterzusatz sowie als Wirkstoff und Bestandteil von pharmazeutischen Produkten von großer industrieller Bedeutung. Für die Herstellung von L-Lysin ist die Fermentation das bedeutendste Verfahren. Hierzu werden neben anderen vor allem Stämme von Corynebacterium glutamicum, Brevibacterium flavum und Brevibacterium lactofermentum verwendet.
Durch Mutationen ist die Regulation der L-Lysin-Biosynthese dieser Stämme so verändert, daß sie L-Lysin über den Eigenbedarf hinaus produzieren und in das Medium ausscheiden.
Eine wichtige Gruppe von L-Lysin ausscheidenden Stämmen sind Homoserin-bedürftige Mutanten der oben genannten Stämme.
Derartige Stämme sind z. B. in der US-PS 3,711,374 beschrieben. Wie dem Fachmann bekannt ist, können derartige Mutanten nach Bestrahlung mit ultraviolettem Licht oder nach Behandlung mit N-Methyl-N'-Nitro-N-Nitrosoguanidin (MNNG) isoliert werden.
Dem Fachmann ist weiterhin bekannt, daß insbesondere bei der Mutagenese mit MNNG Mehrfachmutationen ins Chromosom des Wirtes eingeführt werden können (Guerola, N., Ingraham, J.L. und Cerda-Olmeda, E., Nature 230, 122 bis 125 (1971).
Da sich die nach einer derartigen Mutagenese isolierten Mutanten somit unterscheiden, muß durch ein aufwendiges Suchverfahren die geeignete Mutante identifiziert werden.

Die der vorliegenden Erfindung zugrunde liegende Aufgabe besteht darin, in beliebige Stämme von Aminosäure-ausscheidenden coryneformen Bakterien, z. B. Corynebacterium glutamicum oder Brevibacterium flavum, gezielt eine Homoserin-Auxotrophie einzuführen und mit den erhaltenen Mutanten L-Lysin zu produzieren.

Gegenstand der Erfindung ist ein Verfahren zur Herstellung von L-Lysin durch die an sich bekannte Fermentation von coryneformen Bakterien, insbesondere der Gattungen Corynebacterium oder Brevibacterium, das dadurch gekennzeichnet ist, daß man aufgrund einer Insertionsmutagenese Homoserin Dehydrogenase defekte Mutanten dieser Bakterien einsetzt.
Die Insertionsmutagenese wird gemäß dem bei A. Schäfer et al. (Journal of Bacteriology Vol. 172, Mar 1990, 1663-1666) beschriebenen Verfahren zum konjugativen Transfer von mobilisierbaren Vektoren aus E.coli in Gram-positive Bakterien durchgeführt.
Zu diesem Zweck konstruiert man mobilisierbare, nicht selbsttransferierbare Vektoren, die zusammengesetzt sind aus den folgenden Bestandteilen:
a) ein DNA-Segment, enthaltend ein in E.coli funktionelles Replikon,
b) ein zweites DNA-Segment, enthaltend das für die Mobilisierungsfunktion codierende DNA-Fragment (mob-Region enthaltend ori T),
c) ein drittes DNA-Segment, bei dem es sich in der vorliegenden Erfindung um ein DNA-Fragment eines Homoserin-Dehydrogenase-Gens (hom-Gen) handelt, das in dem zu transformierenden Bakterium homolog mit dem dort befindlichen entsprechenden Gen rekombiniert.

Vorzugsweise wählt man einen inneren Teil des hom-Gens aus Corynebacterium glutamicum ATCC13032, der für die Aminosäuren Nr. 8 (L-Serin)bis Nr. 209 (L-Alanin) der Homoserin Dehydrogenase kodiert.
Die homologe Rekombination führt zur Insertion des Vektors in das Chromosom und damit zur Inaktivierung des entsprechenden Gens.
Dies läßt sich bei einem Vergleich der Enzymaktivität im Ausgangsstamm und in den mutagenisierten Stämmen nachweisen, die aufgrund ihrer Homoserin Auxotrophie leicht zu finden sind.

Es zeigt sich ebenso, daß durch die Integrationsmutagenese von coryneformen Bakterien abgeleitete Homoserin Dehydrogenase defekte Transkonjuganten eine erhöhte Lysinausscheidung zeigen, wenn sie vorher schon als L-Lysinproduzenten bekannt waren, bzw. beginnen, L-Lysin auszuscheiden, wenn sie vorher noch kein L-Lysin produziert haben.

Dazu zählen insbesondere Corynebacterium glutamicum und Brevibacterium flavum.

Die geeigneten mobilisierbaren E.coli Vektoren sind nicht selbsttransferierbar.
Diese Vektoren sind von Plasmiden abgeleitet, die in E.coli-Stämmen selbstständig replizieren und sich für gentechnologische Anwendungen als geeignet erwiesen haben.
Beispiele solcher E.coli Plasmide sind pK18, pK19, pBR322, pBR325, pUC8, pUC9, pUC18, pUC19, pACYC184, pACYC177 oder pLG339.

Diese und andere Vektoren, die nur in E.coli-Stämmen replizieren, werden durch Insertion der mob-Region eines Plasmids mit weitem Wirtsbereich in Gram-negativen Bakterien modifiziert.
Bevorzugt wird das Plasmid RP4 für diesen Zweck verwendet. E.coli Vektoren, die beispielsweise ein 1,9 kb großes Fragment mit der mob-Region enthaltend oriT von RP4 tragen, lassen sich erfindungsgemäß vorteilhaft einsetzen.

Als Mobilisatorstämme geeignet sind insbesondere modifizierte E.coli-Stämme, in deren Chromosom ein RP4-Derivat integriert ist, dessen Transfer-Funktion in trans auf die mob-site der betreffenden Vektoren einwirkt.

Derartige Stämme werden in der US-PS 4,626,504 beschrieben.

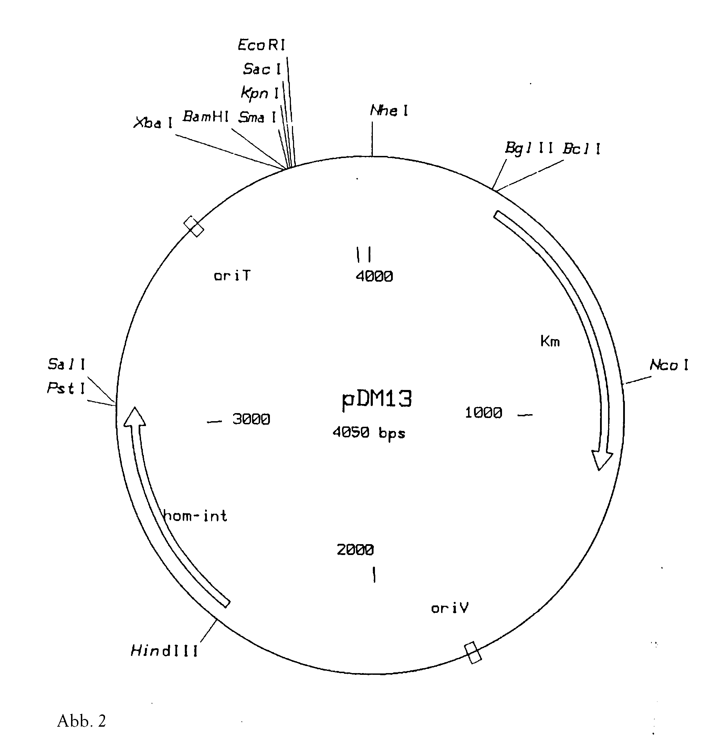

Ein erfindungsgemäß besonders geeigneter Vektor ist pDM13, dessen Konstruktion im Beispielteil beschrieben und dessen Restriktionskarte in Abb. 2 wiedergegeben wird.

### 1. Konstruktion des mobilisierbaren Escherichia coli-Vektors pEM5.2.

Das Plasmid pEM5.2 ist ein Derivat des dem Fachmann bekannten E. coli Kloniervektors pK19 (Pridmore, R.D., Gene 56, 309-312 (1987)). pEM5.2 trägt die für Mobilisierung essentielle Region des selbsttransferierbaren Plasmides RP4 (Datta, N. et al., J. Bacteriology 108, 1244-1249 (1971)) und wurde wie folgt konstruiert: Das Plasmid pSUP102, welches die Mobilisierungsregion (mob) des Plasmides RP4 trägt (Simon, R. et al., Methods in Enzymology 118, 640-659 (1986)), wurde aus E. coli S17-1 (Simon. R. et al., Bio/Technology 1, 784-794 (1983)) nach dem Fachmann geläufigen Verfahren, wie z.B. bei Holmes, D.S. & Quigley, M., (Analytical Biochemistry 114, 193-197 (1981)) beschrieben, isoliert und mit dem Restriktionsenzym TaqI gespalten. Plasmid pK19 wurde mit dem Restriktionsenzym AccI behandelt, mit den durch TaqI-Verdauung des Plasmides pSUP102 erhaltenen DNA-Fragmenten vereinigt und das DNA-Gemisch mit T4-DNA-Ligase behandelt. Kompetente Zellen von Escherichia coli S17-1, die nach der Vorschrift von Cohen, S. et al. (Proceedings of the National Academy of Science USA 69, 2110-2114 (1972)) hergestellt worden waren, wurden mit dem Ligationsgemisch transformiert und die Zellen auf PA-Agar (17,5 g Penassay-Broth (Difco Laboratories), 15 g Agar) mit 25 µg/ml Kanamycin aufgebracht. Kanamycin-resistente Kolonien wurden mit PA-Flüssigmedium abgeschwemmt und in 50 ml PA-Flüssigmedium angezogen und nach gängigen Methoden, wie bei Simon, R. et al. (Bio/Technology 1, 784-794 (1983)) beschrieben, zur Kreuzung mit E. coli MM294(Nx^{R}) eingesetzt. Aus den auf Selektionsmedium (PA-Agar mit 25 µg/ml Kanamycin und 50 µg/ml Nalidixinsäure) gewachsenen Kolonien konnte ein Plasmid von 3,4 kb Länge isoliert werden. Der hier als pEM5.2 bezeichnete Vektor trägt eine DNA-Insertion von 776 bp Länge. Durch DNA-Sequenzanalyse wurde die Nukleotidsequenz des inserierten DNA-Fragments in Plasmid pEM5.2 bestimmt.

Die Nukleotidsequenz ist identisch mit einem Teil der DNA-Sequenz der mob-Region des Plasmids RP4 (Fürste, J.P. et al., Proceedings of the National Academy of Science USA 86, 1771-1775 (1989)). Die Restriktionskarte des Plasmids pEM5.2 ist in Abbildung 1 dargestellt.

### 2. Konstruktion des mobilisierbaren Vektors pDM13 zur Integrationsmutagenese des Homoserin-Dehydrogenase-Gens (hom) von Corynebacterium glutamicum.

Das hom-thrB-Operon von Corynebacterium glutamicum ATCC13032, das für die Enzyme Homoserin-Dehydrogenase und Homoserin-Kinase kodiert, wurde von Follettie, M.T. et al. (Molecular Microbiology 2, 53-62 (1988)) und Peoples. O.P. et al. (Molecular Microbiology 2, 53-62 (1988)) isoliert, beschrieben und sequenziert. Das hom-thrB-Operon liegt auf einem 3685 bp langen DNA-Fragment, das an beiden Enden Erkennungssequenzen für die Restriktionsendonuklease SalI trägt.

Dieses DNA-Fragment wurde aus Corynebacterium glutamicum ATCC13032 isoliert und unter Zuhilfenahme des Plasmidvektors pJC1 (Cremer, J. et al., Journal of General Microbiology 134, 32211-3229 (1988)) in den Escherichia coli-Stamm Gif102 (Thèse, J. and Saint-Girons, I., Journal of Bacteriology 118, 990-998 (1974)) nach dem Fachmann geläufigen Methoden kloniert. Das dabei entstandene Plasmid pJChom2 wurde aus Escherichia coli Gif102/pJChom2 durch CsCl-Dichtegradientenzentrifugation(Maniatis, T. et al. (1982), Molecular Cloning: A Laboratory Manual, Cold Spring Harbor) isoliert. Plasmid pJChom2 wurde mit den Restriktonsenzymen HindIII und PstI behandelt und ein 606 bp langes DNA-Fragment durch Agarosegel-Elektrophorese mit anschließender Elektroelution (Maniatis, T. et al. (1982), Molecular Cloning: A Laboratory Manual, Cold Spring Harbor) isoliert. Das isolierte DNA-Fragment umfaßt einen inneren Teil des hom-Gens, der für die Aminosäuren Nr. 8 (L-Serin) bis Nr. 209 (L-Alanin) der Homoserin-Dehydrogenase kodiert. Plasmid pEM5.2 wurde mit den Restriktionsenzymen HindIII und PstI verdaut und anschließend mit alkalischer Phosphatase behandelt. Die derart behandelte pEM5.2 DNA wurde mit dem oben beschriebenen 605 bp langen HindIII-PstI-DNA-Fragment gemischt und in Gegenwart von ATP mit T4-DNA-Ligase (Maniatis, T. et al (1982)), Molecular Cloning: A Laboratory Manual, Cold Spring Harbor) behandelt. Der Escherichia coli Stamm S17-1 wurde, wie bei Hanahan, D. (Journal of Molecular Biology 166, 557-580 (1983)) beschrieben, kompetent gemacht und mit dem Ligationsgemisch transformiert. Die Transformanten wurden durch Bebrütung bei 37°C auf LB-Agar (bezogen von der Firma Life Technologies G.m.b.H., Eggenstein, BRD), dem Kanamycin mit einer Konzentration von 25 µg/ml hinzugefügt worden war, selektioniert. Plasmid-DNA wurde aus einer Transformante isoliert und zur Charakterisierung mit den Restriktionsenzymen HindIII und PstI verdaut. Die Restriktionskarte des als pDM13 bezeichneten Plasmids ist in Abbildung 2 dargestellt.

### 3. Integrationsmutagenese des Homoserin-Dehydrogenase-Gens (hom) von Corynebacterium glutamicum ATCC13032 und Brevibacterium flavum ATCC14067 mit Hilfe des Plasmids pDM13.

Das mobilisierbare Plasmid pDM13 wurde ausgehend von dem Escherichia coli Donor-Stamm S17-1/pDM13 mit Hilfe der von Schäfer, A. et al. (Journal of Bacteriology 172, 1663-1666)) beschriebenen Konjugationsmethode in die Stämme Corynebacterium glutamicum ATCC13032 und Brevibacterium flavum ATCC14067 als Rezipienten eingeführt und die Transkonjuganten auf LB-Agar, dem Kanamycin (25 µg/ml) und Nalidixinsäure (50 µg/ml) hinzugefügt worden war, selektioniert. Ausgewählte Transkonjuganten wurden einmal auf StandardI-Agar (Merck, Darmstadt. BRD), dem Kanamycin (10 µg/ml) hinzugefügt worden war, vereinzelt. Anschließend wurde geprüft, ob die Transkonjuganten Homoserin-bedürftig waren. Zu diesem Zweck wurden die beiden Rezipienten (ATCC13032 und ATCC14067) und jeweils 20 Transkonjuganten (ATCC13032::pDM13) von Stamm ATCC13032 und 3 (ATCC14067::pDM13) von Stamm ATCC14067 auf Minimalagar (Katsumata. R. et al., Journal of Bacteriology 159, 306-311 (1084)), Minimalagar mit D,L-Homoserin (200 µg/ml), Minimalagar mit L-Methionin (100 µg/ml) und L-Threonin (100 µg/ml) und auf Minimalagar mit L-Methionin, L-Threonin und Kanamycin (10 µg/ml) aufgebracht und das Wachstum nach 24 Stunden Bebrütung bei 30°C begutachtet. Das Ergebnis ist in Tabelle 1 zusammengefaßt. Sämtliche untersuchten Transkonjuganten waren Homoserin-bedürftig.

**Tab. 1:**

| | | | | | |
|---|---|---|---|---|---|
| Homoserin-Auxotrophie in Transkonjuganten (ATCC13032::pDM13 und ATCC14067::pDM13) von Corynebacterium glutamicum ATCC13032 und Brevibacterium flavum ATCC14067. Die Konzentrationen der Zusätze zum Minimalagar betrugen: 200 µg/ml D,L-Homoserin (D,L-Hse), 100 µg/ml L-Threonin (L-Thr), 100 µg/ml L-Methionin (L-Meth) und 10 µg/ml Kanamycin (Km). Zeichenerklärung: + = Wachstum, - = kein Wachstum. | | | | | |

| Stamm | Zusätze zum Minimalagar | | | | |
|---|---|---|---|---|---|
| | ohne Zusätze | +D,L-Hse | +L-Thr | +L-Meth +L-Thr | +Km +L-Meth +L-Thr |
| ATCC13032 | + | + | + | + | + |
| ATCC13032::pDM13¹⁾ | - | + | - | + | + |
| ATCC14067 | + | + | + | + | + |
| ATCC14067::pDM13²⁾ | - | + | - | + | + |

| | | | | | |
|---|---|---|---|---|---|
| ¹⁾ Es wurden 20 | | | | | |
| (²⁾ bzw. 3) unabhängig entstandene Transkonjuganten untersucht. | | | | | |

### 4. Bestimmung der Homoserin-Dehydrogenase-Aktivität der Transkonjuganten ATCC13032::pDM13 und ATCC14067::pDM13.

Die Stämme ATCC13032, zwei Transkonjuganten (TK-Nr. 1 und 2) vom Typ ATCC13032::pDM13, ATCC14067 und zwei Transkonjuganten (TK-Nr. 5 und 6) vom Typ ATCC14067::pDM13 wurden in 500 ml StandardI-Bouillon (Merck, Darmstadt, BRD), die mit 4 g/l Glucose supplementiert worden war, bei 30°C und 200 rpm bis zum Beginn der stationären Wachstumsphase kultiviert und dann geerntet. Die Zellfeuchtmasse wurde anschließend, wie bei O'Regan, M. et al. (Gene 77, 237-251 (1989)) beschrieben, gewaschen und für die Bestimmung der Enzymaktivität aufgeschlossen.

Der nach mechanischem Zellaufschluß erhaltene Rohextrakt wurde gegen einen Puffer, bestehend aus 100 mM Tris·HCl pH 7,0; 0,8 M (NH₄)₂SO₄; 1 mM DTT; 30 % Glyzerin (v/v), dialysiert. Mit der so erhaltenen Enzympräparation wurde gemäß Tabelle 2 direkt über den photometrisch detektierbaren Verbrauch von NADPH die Homoserin-Dehydrogenase-Aktivität bestimmt. Proteinbestimmungen wurden nach der Methode von Lowry et al. (Journal of Biological Chemistry 193, 265-275 (1951)) durchgeführt. Enzymaktivitäten werden als mU/mg Protein entsprechend einem Umsatz von 1 nmol·min⁻¹·mg⁻¹ angegeben.

In den Transkonjuganten ATCC13032::pDM13 und ATCC14067::pDM13 konnte keine Homoserin-Dehydrogenase-Aktivität nachgewiesen werden (Tabelle 3).

**Tab. 2:**

| | | | |
|---|---|---|---|
| Bedingungen des Homoserin-Dehydrogenase Tests, Meßwellenlänge ist 340 nm. | | | |

| Nr. | ¹⁾Substanz | Test-konzentration | Durchführung |
|---|---|---|---|
| 1 | Tris·HCl pH 7,0/DTT | 100 mM/1 mM | |
| 2 | NADPH | 0,2 mM | |
| 3 | Enzympräparation ²⁾ | verschieden | Blindwertwertmessung |
| 4 | D,L-Aspartyl-β-semialdehyd | 4 mM | Start der Homoserin-Dehydrogenase Reaktion |

| | | | |
|---|---|---|---|
| ¹⁾ Gibt die Reihenfolge der Substanz-Zugabe an. | | | |
| ²⁾ Das Enzym ist in dem im Text geschilderten Puffer gelöst. | | | |

**Tab. 3:**

| | | |
|---|---|---|
| Homoserin-Dehydrogenase-Aktivität in Corynebacterium glutamicum ATCC13032, Brevibacterium flavum ATCC14067 und davon durch Integrationsmutagenese abgeleiteten Homoserin-auxotrophen Transkonjuganten. Abkürzungen: TK = Transkonjugante. | | |

| Stamm | | Homoserin-Dehydrogenase-Aktivität (mU/mg Protein) |
|---|---|---|
| ATCC13032 | | 169 |
| ATCC13032::pDM13 | TK-Nr. 1 | 0 |
| | TK-Nr. 2 | 0 |
| | | |
| ATCC14067 | | 138 |
| ATCC14067::pDM13 | TK-Nr. 5 | 0 |
| | TK-Nr. 6 | 0 |

### 5. Bestimmung der L-Lysin-Ausscheidung der Transkonjuganten ATCC13032::pDM13 und ATCC14067::pDM13.

Die Stämme ATCC13032, ATCC14067 und die davon durch Integrationsmutagenese abgeleiteten Homoserin-Dehydrogenase Transkonjuganten vom Typ ATCC13032::pDM13 (TK-Nr. 1 und 2) und ATCC14067::pDM13 (TK-Nr. 5 und 6) wurden für 72 Stunden bei 30°C und 300 Upm in 10 ml Testmedium inkubiert, das in 100 ml-Erlenmeyerkolben vorlag. Die Zusammensetzung des Testmediums ist in Tabelle 4 aufgeführt. Am Ende der Bebrütung wurde die Kultur abzentrifugiert und die Konzentration an L-Lysin im Kulturüberstand bestimmt. Die Konzentrationsbestimmung erfolgte mit Hilfe eines Aminosäureanalysators (Typ LC5000 der Firma Biotronic, Maintal, BRD) durch Ionenaustauschchromatographie und Ninhydrin-Detektion. Das Ergebnis des Versuchs ist in Tabelle 5 dargestellt.

Die Transkonjuganten schieden signifikant mehr L-Lysin aus als die Elternstämme. Weiterhin ist die L-Lysin-Ausscheidung bei unabhängig entstandenen Transkonjuganten eines Stammes (z.B. TK-Nr. 1 und TK-Nr. 2) von Stamm ATCC13032::pDM13) im Rahmen der Meßgenauigkeit identisch.

**Tab. 4:**

| | |
|---|---|
| Zusammensetzung des verwendeten Testmediums. | |

| Stoff | Konzentration im Medium |
|---|---|
| Melasse | 30 g/l |
| Saccharose | 85 g/l |
| Sojabohnenmehl-Hydrolysat | 185 ml/l |
| Ammoniumsulfat | 2,4 g/l |
| Harnstoff | 17 g/l |
| KH₂PO₄ | 0,5 g/l |
| MgSO₄·7H₂O | 0,4 g/l |
| CaCl₂·2H₂O | 10 mg/l |
| FeSO₄·7H₂O | 12 mg/l |
| MnSO₄·7H₂O | 11 mg/l |
| Tri-Natriumcitrat·2H₂O | 0,6 g/l |
| Biotin | 0,3 mg/l |
| Thiaminhydrochlorid | 0,2 mg/l |
| Ammoniakwasser (10 %) ad pH 7 | |

**Tab. 5:**

| | | |
|---|---|---|
| Ausscheidung von L-Lysin durch die durch Integrationsmutagenese hergestellten Stämme ATCC13032::pDM13 und ATCC14067::pDM13 und durch die Elternstämme. Abkürzungen: TK = Transkonjugante. | | |

| Stamm | | Konzentration L-Lysin·HCl (g/l) |
|---|---|---|
| ATCC13032 | | 0,1 |
| ATCC13032::pDM13 | TK-Nr. 1 | 16,3 |
| | TK-Nr. 2 | 15,8 |
| ATCC14067 | | 0,0 |
| ATCC14067::pDM13 | TK-Nr. 5 | 7,9 |
| | TK-Nr. 6 | 7,6 |

### Beschreibung der Abbildungen

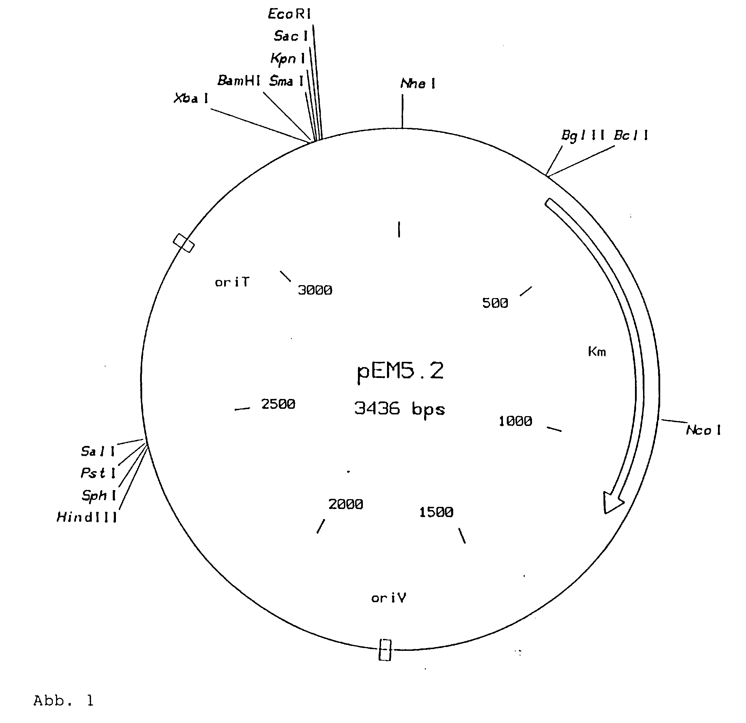

Abb. 1: Restriktionskarte des Plasmids pEM5.2. Das den oriT tragende DNA-Fragment liegt zwischen der SalI-Schnittstelle (Position 2463) und der XbaI-Schnittstelle (Position 3245). Die SalI-Schnittstelle, die mit einer AccI-Schnittstelle identisch ist, entstand durch die AccI/TaqI-Ligation an einer Seite der Insertion neu.
Abkürzungen: Km = Kanamycin-Resistenzgen, oriT = Ursprung der Transferreplikation, oriV = Ursprung der vegetativen Replikation, bps = Basenpaare.

Abb. 2: Restriktionskarte des Plasmids pDM13. Abkürzungen sind in Abb. 1 erlautert.

## Patentansprüche

1. Verfahren zur Herstellung von L-Lysin durch Fermentation von coryneformen Bakterien,
dadurch gekennzeichnet, daß man aufgrund einer Insertionsmutagenese Homoserin Dehydrogenase defekte Mutanten dieser Bakterien einsetzt.

2. Verfahren gemäß Anspruch 1,
dadurch gekennzeichnet, daß man Corynebakterien oder Brevibakterien einsetzt.

3. L-Lysin ausscheidende, durch Insertionsmutagenese Homoserin Dehydrogenase defekte Mutanten coryneformer Bakterien.

4. Bakterien gemäß Anspruch 3,
dadurch gekennzeichnet, daß es sich um Corynebakterien oder Brevibakterien handelt.

5. Bakterien gemäß Anspruch 4,
dadurch gekennzeichnet, daß es sich um Stämme der Arten Corynebacterium glutamicum oder Brevibacterium flavum handelt.

6. Verfahren zur Herstellung von Mutanten gemäß den Ansprüchen 3 bis 5,
dadurch gekennzeichnet, daß man Zellen der zu mutierenden Bakterien mit einem einen mobilisierbaren Vektor tragenden E.coli Mobilisatorstamm kreuzt, wobei in den Vektor ein DNA-Fragment eines Homoserin-Dehydrogenase-Gens (hom-Gen) inseriert ist, das in dem zu transformierenden Bakterium mit dem entsprechenden homologen Gen rekombiniert.

7. Verfahren nach Anspruch 6.
dadurch gekennzeichnet, daß das DNA-Fragment einen inneren Teil des hom-Gens umfaßt, der für die Aminosäuren Nr. 8 (L-Serin) bis Nr. 209 (L-Alanin) der Homoserin Dehydrogenase kodiert und aus Corynebacterium glutamicum ATCC13032 stammt.

8. Mobilisierbarer Vektor pDM13, gekennzeichnet durch die Restriktionskarte gemäß Abb. 2.
und hergestellt aus einem SalI Restriktions 3685 bp langen DNA-Fragment das das hom-Gen trägt und mit Hilfe von pJCl in E. coli kloniert wird, und Behandlung des entstandenen pJChom2 mit HindIII und PstI und Isolierung eines hom-Gen DNA-Fragments; Fragment gemischt mit pEM5.2 und behandelt mit Ligase, E. coli transformiert mit dem Ligationsgemisch, Selektionierung auf Kanamycin und Isolierung von HindIII und PstI Restriktions Transformant-Plasmid-DNA das dem Vektor pDM13 entspricht.

## Claims

1. Method for the production of L-lysine by fermentation of coryneform bacteria, characterised in that mutants of these bacteria which are homoserine dehydrogenase defective owing to an insertion mutagenesis are used.

2. Method according to claim 1, characterised in that Corynebacteria or Brevibacteria are used.

3. L-lysine excreting mutants of coryneform bacteria which are homoserine dehydrogenase defective owing to insertion mutagenesis.

4. Bacteria according to claim 3, characterised in that they are Corynebacteria or Brevibacteria.

5. Bacteria according to claim 4, characterised in that they are strains of the species Corynebacterium glutamicum or Brevibacterium flavum.

6. Method for the production of mutants according to claims 3 to 5, characterised in that cells of the bacteria to be mutated are crossed with an E. coli mobiliser strain carrying a mobilisable vector, a DNA fragment of a homoserine dehydrogenase gene (hom gene) being inserted into the vector, which gene recombines with the corresponding homologous gene in the bacterium to be transformed.

7. Method according to claim 6, characterised in that the DNA fragment contains an inner part of the hom gene, which part codes for the amino acids no. 8 (L-serine) to no. 209 (L-alanine) of the homoserine dehydrogenase and originates from Corynebacterium glutamicum ATCC13032.

8. Mobilisable vector pDM13, characterised by the restriction map according to Figure 2 and produced from an SalI restriction 3685 bp long DNA fragment which carries the hom gene and is cloned in E. coli with the aid of pJC1, and treated with HindIII and PstI of the pJChom2 formed and a hom gene DNA fragment is isolated; the fragment is mixed with pEM5.2 and treated with ligase, E. coli is transformed with the ligation mixture, selected on kanamycin and HindIII and PstI restriction transformant plasmid DNA which corresponds to the vector pDM13 is isolated.

## Revendications

1. Procédé de préparation de L-lysine par fermentation de bactéries coryneformes, caractérisé en ce qu'on utilise des mutants de ces bactéries défectueux en déshydrogénase homosérine en raison d'une mutagenèse par insertion.

2. Procédé selon la revendication 1, caractérisé en ce qu'on utilise des Corynebactéries ou des Brévibactéries.

3. Mutants de bactéries coryneformes défectueux en déshydrogénase homosérine par mutagenèse par insertion, excrétant de la L-lysine.

4. Bactéries selon la revendication 3, caractérisées en ce qu'il s'agit de Corynebactéries ou de Brévibactéries.

5. Bactéries selon la revendication 4, caractérisées en ce qu'il s'agit de souches de types Corynebacterium glutamicum ou Brevibacterium flavum.

6. Procédé de préparation de mutants selon les revendications 3 à 5, caractérisé en ce qu'on croise des cellules des bactéries à faire muter avec une souche mobilisatrice de E. coli portant un vecteur mobilisable, en insérant dans le vecteur un fragment d'ADN d'un gène de déshydrogénase homosérine (gène hom), qui est recombiné avec le gène homologue correspondant dans la bactérie à transformer.

7. Procédé selon la revendication 6, caractérisé en ce que le fragment d'ADN comprend une partie interne du gène hom qui code pour les acides aminés n° 8 (L-serine) au n° 209 (L-alanine) de la déshydrogénase homosérine et est issue de la Corynebacterium glutamicum ATCC13032.

8. Vecteur mobilisable pDM13, caractérisé par la carte de restriction selon la Fig. 2, préparé à partir d'un fragment d'ADN d'une longueur de 3685 bp de restriction SalI, qui porte le gène hom et est cloné à l'aide de pJC1 dans E. coli, le tout étant suivi du traitement du pJChom2 obtenu par HindIII et PstI et isolation d'un fragment d'ADN de gène hom; le fragment étant mélangé à du pEM5.2 et traité avec une ligase; la souche E. coli étant transformée par le mélange de ligature, cette opération étant suivie d'une sélection sur Kanamycine et d'une isolation de l'ADN de plasmide transformant aux restrictions HindIII et PstI, qui correspond au vecteur pDM13.
